# EUROPEAN PATENT SPECIFICATION

(11) **EP 0 283 126 B1**
(45) Date of publication and mention of the grant of the patent: **23.06.1993**
(21) Application number: 88301175.1
(22) Date of filing: 12.02.1988
(51) Int. Cl.: A61M 1/00

(54) **Blood collection device**
Blutsammeleinrichtung
Dispositif de recueil du sang

(30) Priority: 13.02.1987 US 14513
(43) Date of publication of application: 21.09.1988
(73) Proprietor: Sherlock, William, Ashland, Oregon 97520 (US)
(72) Inventor: Sherlock, Hugh Paul, (US)
(74) Representative: Senior, Alan Murray

(56) References cited:
- GB-A- 1 051 852
- US-A- 4 161 179
- US-A- 4 443 220
- US-A- 4 559 035

## Description

This invention relates to a body fluid collection device with a collapsible fluid collection bag and bag stiffener means which are resilient.

It is often desirable during surgery, to collect blood from the patient and subsequently return the collected blood to the same patient. Returning the patient's own blood eliminates or reduces the need for supplying the patient with blood from another person and therefore reduces the danger of transmitting a disease to the patient.

After lung surgery, for example, suction is generally applied to the pleural cavity of the patient by means of a catheter connected to a thoracic or chest drainage unit. Blood and gas flow into the drainage unit during the healing process. The drainage unit and collected blood are eventually discarded. If the patient requires blood, stored blood generally from another person must be infused into the patient. More recently, auxiliary autotransfusion blood collection containers have been employed with chest drainage units which permit reinfusion of the collected blood.

In one case, a non-collapsible blood collection bottle is connected to a chest drainage unit so that suction is applied through the bottle to the patient. The blood collected in the bottle can be used to reinfuse the patient. There are, however, certain disadvantages associated with non-collapsible autotransfusion containers. For example, during reinfusion the rigid bottle must be vented to atmosphere to allow the collected blood to flow into the patient. Thus, air is in contact with the blood and may affect the blood characteristics. Special care must be taken to avoid infusion of air into the patient during reinfusion of blood. Also, an air filter must be used at the vent to prevent possible airborne contamination.

In another arrangement, an auxiliary blood collection device includes a pliable bag having an outer sleeve. The bag is held in an expanded condition by a holder having rigid wire arms extending between the bag and sleeve to maintain the bag open to receive blood under suction from the chest drainage unit. After the bag is filled, it can be removed from the holder and used to reinfuse the patient. One problem with this arrangement is that the volume of the pliable bag varies to a significant extent with different suction forces because the walls of the bag tend to move inwardly, especially between the holder arms. Thus, the indicated amount of blood collected may be inaccurate where the operating suction force in the bag differs from a calibration value.

The prior art document US-A-4161179 shows a vacuum bag which creates its own vacuum when expanded by springs which are integral to it. Upon this document, the prior art portion of claim 1 is based.

It is therefore an object of the present invention to provide an improved blood collection device which permits the collection and reinfusion of the same blood and overcomes the disadvantages mentioned above.

Another object is to provide an improved autotransfusion blood collection device well suited for connection to a chest drainage unit wherein blood collected from the patient can readily be collected for reinfusion in a safe and efficient manner.

According to the invention, there is provided a body fluid collection device comprising a collapsible bag having a pair of opposed sidewalls an inlet for receiving body fluid, stiffener means including a pair of relatively stiff flexible members respectively adjacent said sidewalls and opposed side edges, said members having portions between said side edges bendable outwardly in opposite directions from each other to expand said bag in response to the application of a force external to said bag tending to move said side edges generally toward each other for receiving body fluid through said inlet
characterised in that said members are biased to keep the bag closed and flat in their unstressed state; and
in that the device further comprises means external to said bag for holding the bag and maintaining a compressive force on said opposed side edges to maintain said bag expanded, including a pair of opposed members spaced apart less than the distance between said side edges in the bag's unexpected state for receiving said bag and flexible members therebetween.

Such a bag can be held in the expanded condition by a holder, such as a ring, which maintains the squeezing force on said edges. The bag may have an outlet suitable for use with a standard infusion set to permit immediate reinfusion once the bag is disconnected from the patient drain tube. Once the holder is removed the bag can collapse as blood is reinfused; a standard clamp may be applied to the bag to increase the rate of infusion.

In one preferred embodiment, the flexible members are inside the bag and push the bag walls outward as the squeezing force is applied.

In another preferred embodiment, the flexible members are attached to the outside of the bag.

Preferably, the flexible members are of a sheet material which provides sufficient support to the bag to avoid significant variations in volume when under suction. The flexible members may be joined together along one adjacent side edge in a manner of a hinge and may have slits extending from the upper and lower edges to allow the flexible members to assume more closely the expanded shape of the bag. In a preferred embodiment a symmetrical pair of slits is provided at the upper and lower edge, each pair of slits diverging toward a respective side edge.

The holder for the bag may have means for attachment to a conventional suction drainage unit so that suction can be applied through the bag to the patient.

The blood collection bag assembly may be shipped and stored in a collapsed condition, and expanded for use in collecting blood.

Other features of the invention will be apparent from the following description of two preferred embodiments shown by way of example only in the accompanying drawings in which:-
Fig. 1 is an elevation, partly in section, of a chest drainage system in accordance with a preferred embodiment of the present invention;
Fig. 2 is a cross-section on line 2-2 of Fig.1;
Fig. 3 is a cross-section on line 3-3 of Fig.1;
Fig. 4 is an elevation of the autotransfusion bag assembly of Fig. 1 but in its unrestrained condition;
Fig. 5 is a cross-section on line 5-5 of Fig.4
Fig. 6 is an elevation of the bag stiffening member of Fig.1 but in its unrestrained condition; and
Fig 7 is an elevation of an autotransfusion bag assembly in accordance with a modified embodiment of the present invention.

Referring now to the drawings, a chest drainage system 10 includes autotransfusion blood collection device 12 attached to the side of a chest drainage unit 14. The device 12 includes an autotransfusion bag assembly 16 for blood collection and reinfusion, and a bag expanding member or holder 18 receiving the bag assembly 16. As shown for illustration, the holder 18 has a pair of integral depending lugs 20 and 22 respectively received in a pair of integral brackets 24 and 26 on the chest drainage unit 14 whereby the device 12 is removably connected to the unit 14.

The holder 18 maintains the bag assembly 16 in an expanded condition for receiving body fluids from the patient, as will be further explained below. The bag assembly 16 has a fluid inlet 28 connected through a flexible tube 29 and tube connector 30 to a suction catheter 31 that has its distal end inserted into the plueral cavity of a patient 32, the connector and catheter being illustrated in phantom.

Body fluids, such as air, gas and blood, flow from the patient into the bag assembly 16 during operation of the system 10. The bag assembly 16 also includes a gas outlet 33 connected through a flexible tube 34, connector 37 and flexible tube 38 to the inlet 36 of the chest drainage unit 14.

The chest drainage unit 14 is shown for illustration and includes a fluid collection chamber 40, a liquid seal chamber indicated generally at 42, and a liquid manometer indicated at 44. The liquid seal chamber includes a relatively narrow vertical channel 46 open at the top where it is in fluid communication with the collection chamber 40.

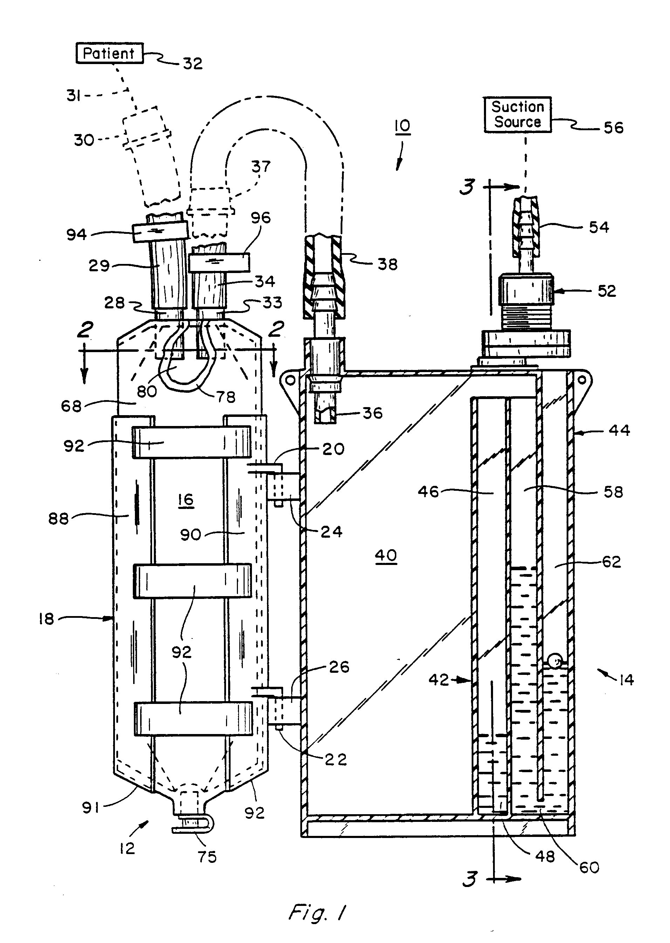

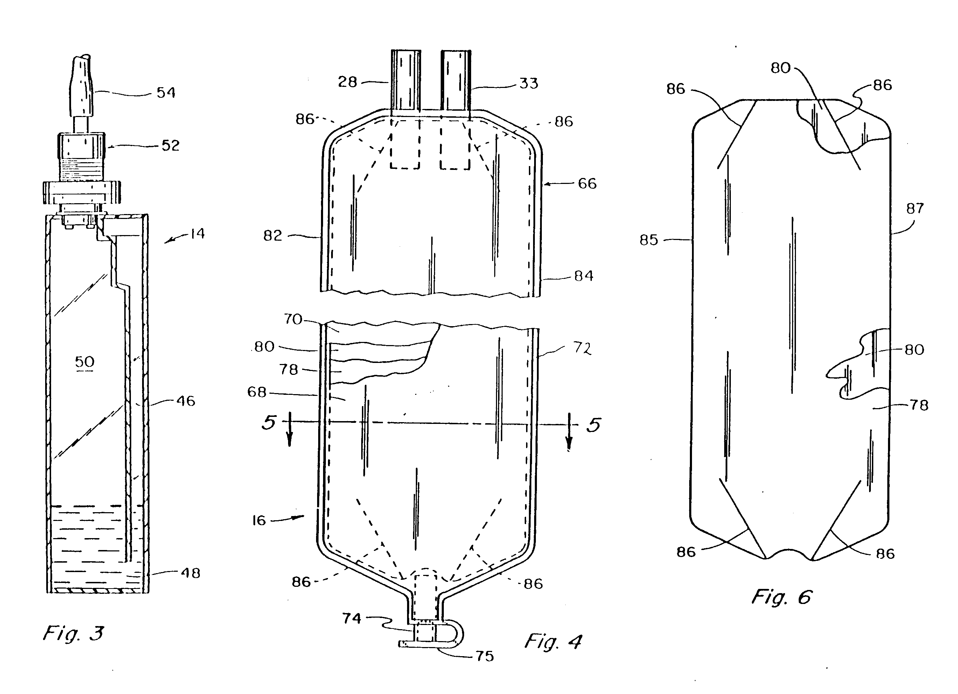

Channel 46 has an opening 48 at the bottom which communicates with a relatively large gas outlet chamber 50 as seen in Fig. 3. Connected to the top of outlet chamber 50 is a suction regulator 52 that is connected by a flexible tube 54 to a suitable source of suction 56 (Fig. 1). The liquid manometer includes a vertical channel 58 in fluid communication with collection chamber 40 at the top, and by a passage 60 at the bottom to a second vertical channel 62 that is open at the top to atmosphere. Both the seal chamber 42 and the manometer 45 are shown with liquid therein.

When a partial vacuum exists in the outlet chamber 50(Fig 3), any air or gas from the patient flows from catheter 31 through the upper portion of bag assembly 16 and through outlet 33 into collection chamber 40 where it bubbles through the liquid in channel 46, through the bottom opening 48 and through the outlet chamber 50 to the suction source 56. The liquid seal 42 prevents any atmospheric air from flowing through the unit 14 to the patient. Because the manometer 44 is responsive to the pressure in collection chamber 40, the level of liquid therein will provide an indication of the negative pressure in the pleural cavity of the patient. The construction and operation of the chest drainage unit 14 including the suction regulator 52 are shown and described in detail in U.S. patent No. 4,372,336.

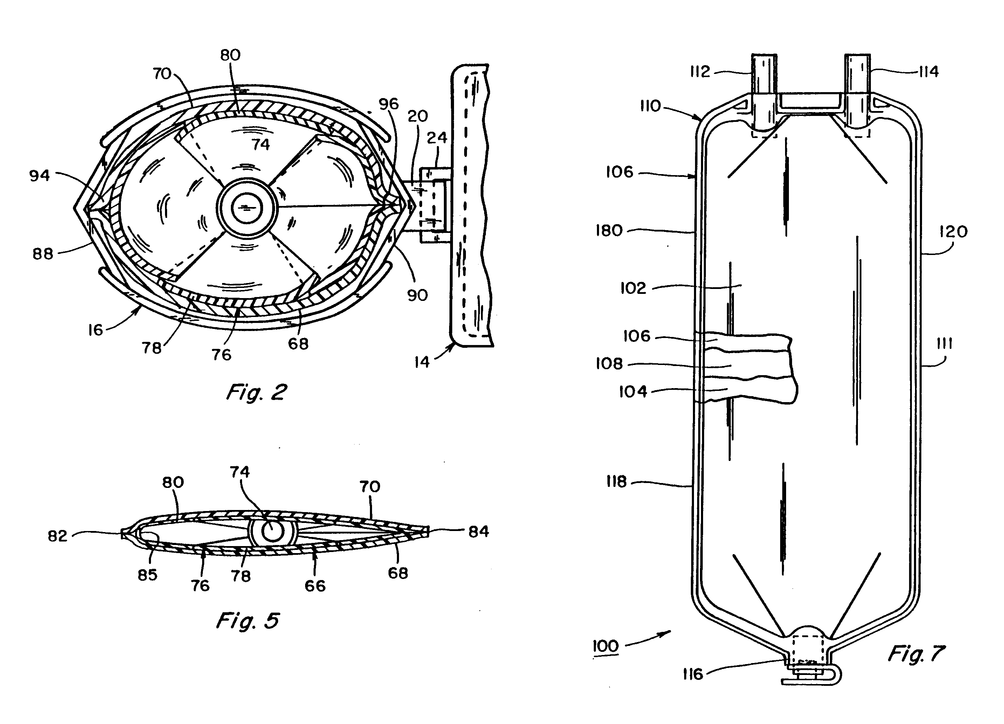

The collection and reinfusion bag assembly 16, shown also in its unrestrained or free condition in Figs. 4 and 5, includes a collapsible bag 66 formed of two opposed pliable walls 68 and 70 connected together, for example by heat sealing, to effect a peripheral seal indicated at 72. The peripheral seal 72 extends around the inlet 28 and outlet 33 to seal them to the bag 66. Also, the seal 72 extends around and seals a tubular blood outlet 74 at the bottom of the bag 62. A closure plug 75 integrally tethered to the bag normally maintains the outlet 74 closed.

The bag assembly 16 also includes a resilient bag stiffener indicated generally at 76 which maintains the pliable bag 66 in an expanded condition when the assembly 16 is in the holder 18. The stiffener 76, which is shown also in its unrestrained or free condition in Figs. 5 and 6, is formed of a pair of parallel facing sheet members or panels 78 and 80 shown, for example, integrally connected at one side 82 and secured together such as by an adhesive or heat seal at the opposite side 84. Stiffener members 78 and 80 are made of a flexible but relatively rigid plastic, they being preferably substantially more rigid and resilient than the walls 68 and 70 of bag 66. For example, the bag 66 may be formed of a pliable plastic such as a pliable polyvinyl chloride while the stiffener members 78 and 80 may be formed of a flexible, more resilient and rigid plastic such as polyethylene terephthalate (PET-G).

While the stiffener members 78 and 80 may be discrete sheets they are shown within bag 66 as integrally connected at the left side 82, the left side serving as a hinge. Stiffener members 78 and 80 may be cut from sheet stock or moulded in sheet form.

The stiffener 76 may be folded at one side and seamed at the other so that there is a slight outwardly bowing as shown in Fig.5.

This outward bowing facilitates proper bending of the stiffener members to produce a rounded bag assembly (Fig.2) when compressive forces are applied at the opposed sides 82 and 84, as will be further discussed below. Also, each of the members 78 and 80 is provided as its upper and lower ends with a pair of slits 86 that extend inwardly.

While the slits 86 may be vertical, each pair of slits is shown to diverge as they extend inwardly from the end edge of the member, the slits of each pair being closest to each other at the mouth. The slits 86 allow adjacent portions of the stiffener members to move past each other (Fig. 2) tending to allow the bag 66 to generally become tapered at the top and conical at the bottom when the opposed sides 85 and 86 of the stiffener 76 are urged toward each other as compressive forces are applied to the opposed sides 82 and 84. The slits 86 may be vertical and still aid in allowing the opposed ends of the bag assembly to take a satisfactory shape. The side edges 82 and 84 of the bag are respectively adjacent side edges 85 and 86 of the stiffener 76.

The holder 18 may be of various constructions, so long as it holds the bag assembly 16 in an expanded condition as illustrated in Figs. 1 and 2. The holder 18 includes two opposed rigid vertical members 88 and 90 secured together by connecting struts 92 and maintained in predetermined spaced relation from each other.

As best seen in Fig. 2, the members 88 and 90 are V-shaped and the lateral distance between the inside corners of these members is less than the width of the bag assembly 16, including the width of the stiffener member 76. That is, the distance between the inside corners of the holder members is less than the distance between the side edges 82 and 84 of bag assembly 16, when in the uncompressed or generally flattened state as in Figs. 4 and 5.

In this way, when clamping or compressive forces are applied to the opposite sides 82 and 84 of the assembly 16 compressive forces are applied to side edges 85 and 86 of the stiffener members 78 and 80 so that central portions of members 78 and 80 bow outwardly causing the pliable walls 68 and 70 of bag 66 to follow and expand to the condition shown in Figs. 1 and 2.

When inserting bag assembly 16 into the holders 18, opposed compressive forces are applied to sides 82 and 84 urging these sides toward each other as well as urging sides 85 and 86 of the stiffener 76 together; the stiffener members 78 and 80 oppositely bow and expand the bag assembly 16 including bag 66. While in the expanded condition, the bag assembly 16 is inserted into the upper open end of holder 18 with the opposed sides 82 and 84 of the bag assembly received in the inside corners of the V-shaped holder members 88 and 90 for proper orientation of the bag 66 and holder. The bag assembly 16 is slid downwardly until it engages a bottom walls 91 and 92 (Fig. 1) of the holder 18. Since the holder members 88 and 90 are spaced from each a distance less than the normal or free width of the bag assembly 16 (Fig. 4), the bag 16 is held open by the holder.

The bag assembly 16 is held in an approximately cylindrical shape in the holder 18 which shape provides the greatest resistance to collapse from negataive pressure and provides a desired volume for blood collection.

With the autotransfusion device 12 connected and arranged as shown in Fig.1, fluid from the patient flows through tube 30 into the expanded bag assembly 66. Blood flows toward the bottom of the bag assembly while suction applied through the chest drainage unit causes gas from the patient to pass through the gas outlet 33 to the suction source 56 as previously described. Thus the blood and gas from the patient are separated, the blood filling the bag 66 and gas being removed through the chest drainage unit. Should the bag assembly 66 fill with blood, the excess will overflow into the collection chamber 40 by way of gas outlet 33 and tube 38. Preferably, the autotransfusion device 12 is replaced by an empty unit before it is full.

In use, after the autotransfusion bag assembly 16 has been filled with the patients blood to a desired level, the tubes 29 and 34 may be closed by suitable tube clamps, such as those indicated at 94 and 96 in Fig. 1, to allow the catheter 31 and inconnecting tube 38 to be removed from the tube connectors 30 and 37. The bag assembly 16 can then be slid upwardly and out of holder 18 so that blood collected can be returned to the same patient. The plug 76 can be removed and infusion tubes connected to the blood outlet 74 for reinfusing the blood into the patient.

Since the bag assembly 16, when removed from holder 18, is collapsible, the bag assembly 16 can conveniently be used in the same or similar manner during infusion as a conventional blood bag. Also, a standard pressure cuff can be used if desired to squeeze the bag 66 where increased blood flow to the patient is desired.

In the modified embodiment shown in Fig. 7, a collection and reinfusion bag assembly 100 is shown including separate stiffening members, indicated at 102 and 104 that are connected such as by an adhesive to the outer surfaces of a pair of opposed pliable walls 106 and 108 of a pliable bag indicated at 110. The assembly 100 is shown in its unrestrained or free state condition. Stiffening members 102 and 104 may be made of a relatively resilient, flexible plastic. Bag 110, like bag 66, has its opposed walls 106 and 108 connected together around the periphery by a peripheral seal 111 which also extends about an inlet 112, gas outlet 114, and a blood outlet 116. When clamping or compressive forces are applied to the opposed sides indicated at 118 and 120, which are adjacent the sides of seam 111, the relatively stiff members 102 and 104 bow outwardly generally into a configuration somewhat circular in section and in doing so cause the adjacent walls 106 and 108 of the pliable bag to follow thereby expanding the pliable bag 110. The expanded bag assembly 100 may then be moved into a suitable holder such as holder 18 in Fig. 1 by which it will be maintained in an expanded condition to receive drainage blood. Bag assembly 100 can be used to reinfuse the patient's blood in the same manner as bag assembly 16.

By employing a pliable blood collection bag having pliable sidewalls or opposed panel members, such as bags 66 or 110, and relatively rigid and flexible opposed sheet or panel members of a different material, the pliable bag is readily maintained in an expanded condition within a holder for efficiently receiving quantities of blood.
Conventional blood infusion techniques can be employed when using the autotransfusion bag assemblies 16 and 100.

## Claims

1. A body fluid collection device (16) comprising a collapsible bag (66) having a pair of opposed sidewalls (68,70), an inlet (28) for receiving body fluid, resilient bag stiffener means (76) including a pair of relatively stiff flexible members (78,80) respectively adjacent said sidewalls (68,70), and opposed side edges, (82,84), said members (78,80) having portions between said side edges bendable outwardly in opposite directions from each other to expand said bag (66), in response to the application of a force external to said bag tending to move said side edges (82,84) generally toward each other for receiving body fluid through said inlet (28);
characterised in that when said flexible members (78,80) are in their unstressed state the bag is flat and biased against expansion by the flexible members; and
in that the device further comprises means (18) external to said bag for holding the bag and maintaining a compressive force on said opposed side edges to maintain said bag expanded, including a pair of opposed members (88,90) spaced apart less than the distance between said side edges (82,84) in the bag's unexpanded state for receiving said bag (66) and flexible members (78,80) therebetween.

2. The device according to claim 1, wherein the flexible members (78,80) are of sheet material.

3. The device according to claim 2,wherein the flexible members (78,80) comprise a pair of sheets connected along one adjacent side edge (82).

4. A device according to any preceding claim, wherein the flexible members (78,80) are of plastic.

5. A device according to any one of claims 3 to 4, wherein the flexible members are generally rectangular and have pairs of slits (86) extending from the upper and lower edges, each pair of slits being symmetrical and diverging from the edge.

6. A device according to any preceding claim, including means (20,22) for attaching the said means (18) for maintaining compressive force to a chest drainage unit, said bag having an outlet (33) for connection to the collection chamber of said unit.

7. A device according to any preceding claim, wherein said means (18) for maintaining compressive force includes a ring.

## Patentansprüche

1. Sammelvorrichtung (16) für Körperflüssigkeiten, umfassend einen zusammenlegbaren Beutel (66), der ein Paar gegenüberliegender Seitenwände (68,70), einen Einlaß (28) zum Aufnehmen der Körperflüssigkeit, elastische Beutelaussteifungsmittel (76), die ein Paar relativ steife flexible Elemente (78,80) enthalten und die jeweils an die Seitenwände (68,70) angrenzen, und gegenüberliegende Seitenkanten (82,84) aufweist, wobei die Elemente (78,80) zwischen den Seitenkanten Teile aufweisen, die in entgegengesetzten Richtungen voneinander nach außen biegbar sind, um den Beutel (66) als Reaktion auf die Anwendung einer externen Kraft auf den Beutel aufzuweiten, wodurch sich die Seitenkanten (82,84) aufeinander zubewegen, um die Körperflüssigkeit durch den Einlaß (28) aufzunehmen,
dadurch gekennzeichnet, daß, wenn die flexiblen Elemente (78,80) in entspanntem Zustand sind, der Beutel flach und gegen die Aufweitung durch die flexiblen Elemente gerichtet ist, und
daß die Vorrichtung weiterhin Mittel (18) umfaßt, die sich außerhalb des Beutels befinden, um den Beutel zu halten und um eine Druckkraft auf die gegenüberliegenden Seitenkanten aufrechtzuerhalten, damit der Beutel aufgeweitet bleibt, wobei diese Vorrichtung ein Paar gegenüberliegende Elemente (88,90) umfaßt, deren Abstand voneinander geringer ist als der Abstand zwischen den Seitenkanten (82,84) im nichtaufgeweiteten Zustand des Beutels, um den Beutel (66) und die flexiblen Elemente (78,80) dazwischen aufzunehmen.

2. Vorrichtung nach Anspruch 1, worin die flexiblen Elemente (78,80) aus einem blattförmigen Material bestehen.

3. Vorrichtung nach Anspruch 2, worin die flexiblen Elemente (78,80) ein Paar dünne Platten umfassen, die entlang einer angrenzenden Seitenkante (82) verbunden sind.

4. Vorrichtung nach einem der vorhergehenden Ansprüche, worin die flexiblen Elemente (78,80) aus Kunststoff sind.

5. Vorrichtung nach einem der Ansprüche 3 bis 4, worin die flexiblen Elemente im allgemeinen rechtwinklig angeordnet sind und ein Paar Schlitze (86) aufweisen, die sich von den oberen zu den unteren Kanten erstrecken, wobei jedes Schlitzpaar symmetrisch ist und von der Kante aus divergiert.

6. Vorrichtung nach einem der vorhergehenden Ansprüche, umfassend Vorrichtungen (20,22) zum Anbringen der Mittel (18) zum Aufrechterhalten der Druckkraft auf eine Brustdrainageeinheit, wobei der Beutel einen Auslaß (33) zur Verbindung mit der Sammelkammer dieser Einheit aufweist.

7. Vorrichtung nach einem der vorhergehenden Ansprüche, worin die Mittel (18) zum Aufrechterhalten der Druckkraft einen Ring umfassen.

## Revendications

1. Dispositif de collecte d'un fluide corporel (16) comportant un sac déformable (66) possédant une paire de parois latérales opposées (68, 70), un orifice d'admission (28) pour recevoir un fluide corporel, des moyens de renforcement (76) du sac élastique comprenant une paire d'éléments flexibles relativement rigides (78, 80) respectivement adjacents auxdites parois latérales (68, 70), et des bords latéraux opposés (82, 84), lesdits éléments (78, 80) possédant des parties entre lesdits bords latéraux pouvant être courbées vers l'extérieur dans des directions opposées l'une par rapport à l'autre pour dilater ledit sac (66), en réponse à l'application d'une force extérieure audit sac tendant à déplacer lesdits bords latéraux (82, 84) généralement l'un vers l'autre pour recevoir un fluide corporel par l'intermédiaire dudit orifice d'admission (28) ;
caractérisé en ce que, lorsque lesdits éléments flexibles (78, 80) se trouvent dans leur état de repos, le sac est plat et sollicité à l'encontre d'une dilatation par les éléments flexibles ; et
en ce que le dispositif comporte en outre des moyens (18) extérieurs audit sac pour tenir le sac et maintenir une force de compression sur lesdits bords latéraux opposés pour maintenir ledit sac dilaté, comprenant une paire d'éléments opposés (88, 90) espacés de moins de la distance entre lesdits bords latéraux (82, 84) dans l'état non dilaté du sac pour recevoir ledit sac (66) et lesdits éléments flexibles (78, 80) entre eux.

2. Dispositif selon la revendication 1, dans lequel les éléments flexibles (78, 80) sont en un matériau en feuille.

3. Dispositif selon la revendication 2, dans lequel les éléments flexibles (78, 80) comportent une paire de feuilles réunies le long d'un bord latéral adjacent (82).

4. Dispositif selon l'une quelconque des revendications précédentes, dans lequel les éléments flexibles (78, 80) sont en matière plastique.

5. Dispositif selon l'une quelconque des revendications 3 ou 4, dans lequel les éléments flexibles sont généralement rectangulaires, et possèdent des paires de fentes (86) s'étendant depuis les bords supérieur et inférieur , chaque paire de fentes étant symétrique et s'écartant du bord.

6. Dispositif selon l'une quelconque des revendications précédentes, comprenant des moyens (20, 22) pour fixer lesdits moyens (18) pour maintenir une force de compression sur une unité de drainage de poitrine, ledit sac possédant un orifice de sortie (33) pour une connexion avec la chambre de collecte de ladite unité.

7. Dispositif selon l'une quelconque des revendications précédentes, dans lequel lesdits moyens (18) pour maintenir une force de compression comprennent une bague.
